# EUROPEAN PATENT SPECIFICATION

(11) **EP 2 823 785 B1**
(45) Date of publication and mention of the grant of the patent: **28.02.2018**
(21) Application number: 13175909.4
(22) Date of filing: 10.07.2013
(51) Int. Cl.: A61C 1/00, A61N 5/06, A61N 5/067

(54) **Handheld laser device for medical purposes**
Tragbare Laservorrichtung für medizinische Zwecke
Dispositif laser portatif à des fins médicales

(43) Date of publication of application: 14.01.2015
(73) Proprietor: Fotona d.o.o., 1000 Ljubljana (SI)
(72) Inventor: Marincek, Marko, 1000 Ljubljana (SI); Perhavec, Tadej, 6210 Sezana (SI); Leskovar, Marko, 4208 Sencurr (SI); Zabkar, Janez, 1230 Domzale (SI)
(74) Representative: Hess, Peter K. G.

(56) References cited:
- EP-A1- 0 626 229
- WO-A-2014/066489
- DE-A1- 19 533 350
- US-A- 4 608 980
- US-A1- 2008 219 629

## Description

### 1. Field of the invention

The present invention relates to a handheld laser device for medical, in particular dental treatment purposes, according to claim 1.

### 2. Technical Background

General considerations of the design of such laser devices are related to compactness, weight, electrical safety and sterility. Commonly the handpiece of such laser devices consists of a central elongated, e. g. tubular housing accommodating the laser and the corresponding auxiliary equipment, e. g. a solid state crystal rod, a flash lamp for optically pumping the rod in a pumping chamber and a water cooling for keeping the temperature of the handpiece on a tolerable level. The cooling water supply and the electrical connections are preferably bundled in form of a cable leaving the tubular housing at one end, whereas the generated laser beam exits the tubular housing at the other end, i.e. the tip end. It is also known in the art to employ a further cylindrical or conical housing which is removable or replaceable, and thus facilitates sterilization and patient's protection.

The US 5,401,171 discloses a dental laser apparatus wherein the laser energy is generated within a handheld handpiece. When the laser is activated, pulsed laser energy is focused by a lens, also situated within the handpiece, onto the tissues in the mouth of a patient. With this device, for hygienic purposes, the whole handheld handpiece has to be sterilized, which is cumbersome and technically problematic since all the electric equipment as, for example the laser generating means, are likewise provided inside of the handpiece.

From US patent 5,548,604 there is known a compact hand held medical laser device having a first housing, a laser cavity situated therein, and a selectively removable second housing removably attached to the first housing having optical beam shaping and beam delivery elements arranged therein. The apparatus uses a solid state crystal laser material rod and a lamp within a cavity. A coolant fluid is arranged to flow therebetween through a folded path having a fluid entrance and fluid exit at the same end of the first housing.

WO 2010/049114 discloses a compact laser device for medical use, having a grippable enclosure connected to an interchangeable tip that emits a working laser beam. The housing of the device is curved downwardly, which facilitates the treatment on the lower jaw of a patient. However, if treatment of e.g. the upper jaw is necessary, the operator has to rotate the device by 180° in an upside down orientation, in which case the device is awkward to handle. EP0626229 and US2008/0219629 also disclose detachable dental laser handpieces.

Based on the known prior art, it is therefore an object of the present invention to provide a handheld laser device for medical purposes which offers a facilitated and safer sterilization, and which at the same time is very ergonomic in use. These and other objects, which become apparent upon the reading the following description, are solved by a handheld laser device according to claim 1.

### 3. Detailed description of the invention

The invention is as defined in claim 1.

According to the invention, a handheld laser device for medical and in particular for dental treatment purposes, is provided, which comprises a handpiece having a handpiece body accommodating a laser and a removable handpiece attachment. The handpiece attachment can be removed to allow sterilization of the attachment for hygienic purposes. Preferably, the removable handpiece attachment is slidable over at least a major portion, i.e. more than 50 % of the length of the handpiece body, preferably more than 60 % and most preferred more than 75 %, so that basically all parts of the handheld laser device, which may come into contact with tissue of a patient, are covered by the handpiece attachment, so that hygienic operating of the device is possible by only sterilizing the handpiece attachment.

The handpiece attachment is preferably a hollow tubular member and comparably inexpensive, since the handpiece body accommodates all the expensive parts of the device, as in particular the laser equipment. It is therefore possible to treat a large number of patients in short time with the same handheld laser device, since only the handpiece attachment has to be exchanged and sterilized, while the handpiece body can be used in a number of operations without the need for a time consuming sterilization process. Thus, for example, a dentist can have a number of handpiece attachments in stock, but only needs one handpiece body, which allows a particular economic operation of the device.

It is evident that sliding the handpiece attachment over the handpiece body in accordance with the present invention can further improve electrical safety, since a further layer, i.e. the handpiece attachment, which is preferably made of a material, which is suitable for medical purposes, such as in particular a titanium alloy, is arranged around the handpiece body containing the electrical components. Sterilization is also facilitated, since the handpiece attachment, which comes in contact with the patient's body tissue and may be contaminated, can be separated completely from the handpiece body.

To allow a facilitated and easy exchange of the handpiece attachment, the same is coupled to the handpiece body by means of latch-type fixation. Therefore, the operator can exchange the handpiece attachment in a very short time without any special tools and without any technical training to do so.

Preferably, the handpiece attachment is adapted to be rotatable relative to the handpiece body, to allow at least two different angular working rotations. This means e.g. that the handpiece attachment can be coupled to the handpiece body in two different angular positions, in particular offset by 180°. Thereby, it is possible to use the device e.g. in an ergonomic manner for the treatment of dental problems in the lower jaw of a patient and - by simply rotating the attachment by 180° around the longitudinal axis of the device - for the treatment of the upper jaw. In a preferred embodiment, the rotating of the attachment relative to the handpiece body is possible in coupled condition of attachment and handpiece. This allows e.g. a particular hygienic operation of the device. However, according to the invention, it is likewise possible to detach the handpiece attachment from the handpiece body, rotate it into the desired angular position, and attach it again. Also in this case the attachment is adapted to be rotatable relative to the handpiece body, to allow at least two different angular working orientations. In any case, it is generally preferred that the laser beam emits essentially at a right angle to the longitudinal extension of the handheld laser device.

According to the invention, a coolant fluid, in particular air and/or water, is supplied from the handpiece body to the handpiece attachment. Thereby, it is possible to guide a coolant fluid, preferably a spray of air and water, onto the spot where the laser beam is focused. Since the air and/or water is supplied from the handpiece body to the attachment, all the supply tubing for air and water can be arranged in or on the handpiece body and needs not to be separately coupled and decoupled when the handpiece attachment is exchanged for the next patient or rotated to a different angular working orientation. Accordingly, it is generally preferred that the coupling of the coolant supply from the handpiece body to the attachment is adapted to work in any angular orientation. This is preferably achieved by employing an annular groove in fluid communication between both of the respective conduits or tubing inside of handpiece body and handpiece attachment. Details of such a coupling mechanism are explained below with reference to the figures.

As mentioned, the advantages of the present invention are in particular realizable by using a particular radial way quick-connection for supplying air and/or water from the handpiece body to the handpiece attachment. As it will be explained in more detail in the following, the radial way connection for supplying air / water allows using the handpiece attachment in different angular orientations with regard to the handpiece body, without interrupting the air / water supply. Thus, since the coolant exit is in or at the handpiece attachment (and not the handpiece body), it is always and automatically directed to the spot of the laser beam, independent of the angular working orientation of the handpiece attachment. Most preferably, the radial way quick-connection for supplying air and/or water from the handpiece body to the handpiece attachment enables the supply of coolant to the attachment in each angular working orientation.

Generally preferred, the handpiece body has an elongated tubular form and preferably comprises a focusing lens at its tip end. As the skilled person recognizes, the tip end is the end of the device from which the laser beam is emitted and - as it is commonly the case - the opposite end of the handpiece body is provided with the necessary energy supply, water supply etc. in form of a suitable cable assembly. Further, the handpiece attachment is provided with a mirror arrangement, and the focusing lens is adapted to interact therewith. Preferably, the handpiece attachment further comprises a corresponding elongated tubular interior cavity adapted to receive the major portion of the handpiece body therein. Most preferably, the handpiece attachment completely encases said major portion of the handpiece body, so that no part of the handpiece body can come into contact with tissue of a patient.

In a preferred embodiment, the handpiece body comprises further a prism which is arranged adjacent to and being adapted to cooperate with the focusing lens, in order to guide the laser beam onto the mirror arrangement. The mirror arrangement is preferably provided in the handpiece attachment and arranged such that it is in the center axis of the handpiece attachment, like for example in the center axis of the tubular interior cavity. This arrangement allows rotating the handpiece attachment relative to the handpiece body, to allow different angular working orientations of the laser: since the mirror arrangement is in the center axis of the tubular interior cavity it is possible to project the laser beam from the handpiece onto said mirror, independent of the angular orientation of the handpiece attachment relative to the handpiece body. Thereby, it is advantageously possible to simply adapt the radial emitter's point of the laser beam by rotating the handpiece attachment with regard to the handpiece body. As mentioned above, this can be achieved by detaching the handpiece attachment and coupling it again in a different angular position or preferably by rotating the handpiece attachment while it is coupled to the body, by means of e.g. a suitable coupling mechanism. Details of such a coupling mechanism are explained in connection with the description of the figures. In any case, due to the advantageous particular radial way quick-connection for air and/or water supply from the handpiece body to the handpiece attachment it is always possible to direct coolant onto the spot where the laser beam is focused, irrespective of the angular position of the handpiece attachment relative to the handpiece body. The handpiece body always stays in the same orientation and needs not to be rotated itself during operation. A rotation of the handpiece body itself is problematic, since the handpiece body is also usually provided with the necessary cabling for energy and cooling supply. The bundling of the necessary cabling is very rigid so that a rotation in the longitudinal direction of the handpiece body is difficult, since the rigid cable bundle is not easily twisted.

### 4. Detailed description of exemplary embodiments

In the following, the invention will be described exemplarily with reference to the enclosed figures, in which
Fig. 1 shows a handheld laser device according to the invention before the removable handpiece attachment is fixed or coupled to the handpiece body;
Fig. 2a and 2b show different cut views of handpiece body and handpiece attachment in coupled condition;
Fig. 3 shows a schematic cut-view of the handpiece body;
Fig. 4 shows again a detail of the interior of the handpiece body when cut perpendicular to the longitudinal direction thereof;
Fig. 5 shows a detail of the coupling mechanism of the handpiece body;
Fig. 6 shows a schematic view of a spring used in the coupling mechanism;
Fig. 7 is a three-dimensional cut view of the laser device showing details of the coupling mechanism; and
Fig. 8 shows a detail of Fig. 7 without the handpiece attachment.

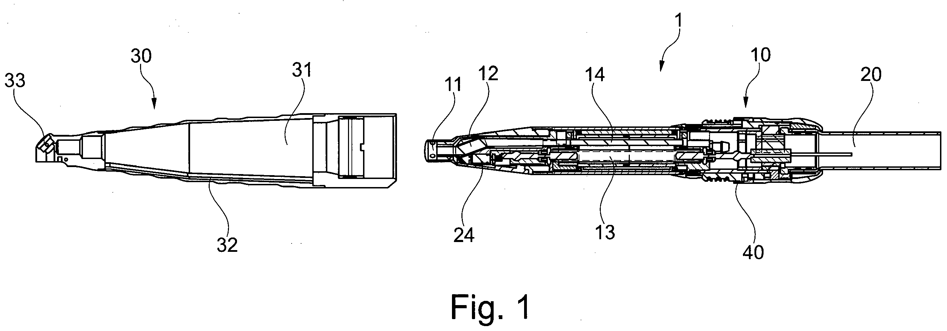

Fig. 1 shows a handheld device 1 in accordance with the present invention in a longitudinally-cut side view. The handheld laser device comprises a handpiece body 10, which is on one end provided with a rigid cable 20 which bundles the different supply conduits, like for example electrical supply conduits or cooling water supply conduits (not shown). The handpiece body has an essentially tubular form and comprises a focusing lens 11 at its tip end. Further, the handpiece body accommodates a laser and the necessary auxiliary equipment to operate the laser. Arranged adjacent the focusing lens 11 a prism 12 is arranged, which is adapted to cooperate with said focusing lens, to guide the laser beam in a direction essentially parallel and/or coaxial to the longitudinally extension of the handpiece 10, so that the laser beam emits out of the tip end through lens 11. The skilled person will understand that the expression "adjacent" in this context means close enough such that the desired cooperation is possible.

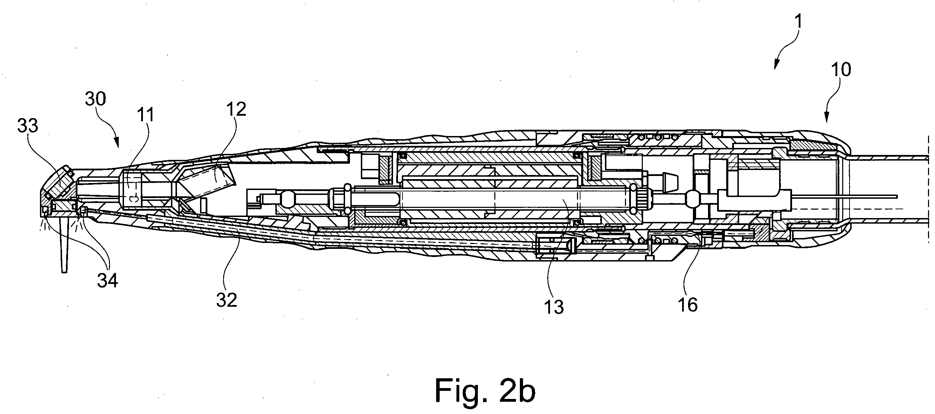

The handpiece attachment 30 is a removable and exchangeable part made preferably from titanium or a titanium alloy. In the view of Fig. 1 the attachment is not slid over the handpiece body. As one can see, the handpiece attachment has an elongated tubular interior cavity, which is adapted to coaxially receive a major portion of handpiece body 10 therein. Extending essentially parallel to the cavity 31, tubing 32 is provided to supply coolant, i.e. air and/or water, to the tip end of handpiece attachment 30. The tubing 32 exits close to the tip end of attachment 30, so that cooling fluid can be discharged onto the spot where the laser beam is focused. The attachment 30 further comprises a mirror arrangement 33 at its tip end. In the shown embodiment, the mirror arrangement 33 deflects the laser beam emitted through lens 11 essentially at a right angle to the longitudinal extension of the handheld laser device 1. In the configuration shown in Fig. 1, the laser beam thus exits downwardly in the figure. According to the invention, it is possible to rotate the attachment 30 with respect to the handpiece body 10, so that it is for example possible to rotate attachment 30 by 180°, so that the deflected laser beam will exit "upside" in the orientation shown in Fig. 1. Thereby, it is easily possible to for example treat the lower jaw of a patient and - upon rotating the attachment 30 by 180° - also the upper jaw, without the need for twisting the handpiece body and the rigid cable 20 attached thereto. This allows a very ergonomic operation of the device 1. Since the tubing 32 and the corresponding exit for the coolant is likewise rotated by 180° when the attachment 30 is rotated (cf. also to Fig. 2b), the coolant fluid can always be directed to the spot where the laser beam is focused without any additional readjusting of the device.

As it can further be taken from the figures, the handpiece attachment 30 completely surrounds and encases a major portion (i.e. preferably more than 50 % of the length of the handpiece body, more preferably more than 60 % of the length and most preferably more than 75 %), so that the only part of the handheld laser device, which can come into contact with tissue of a patient is the removable handpiece attachment 30. Preferably, as e.g. shown in the embodiment, no electrical or electronic equipment is provided in the removable handpiece attachment, so that the whole attachment 30 is relatively inexpensive to produce, at least in relation to the handpiece body 10, and it can withstand the rough conditions necessary for a thorough sterilization. In the shown device, the handpiece attachment 30 is quickly fixed or coupled to the handpiece body by means of a latch-type fixation mechanism 40, which will be described in more detail below. Alternatively, also a bayonet fixation could be feasible. However, a latch-type fixation is preferred, since it facilitates the rotation of the attachment 30 around handpiece body 10 in coupled condition.

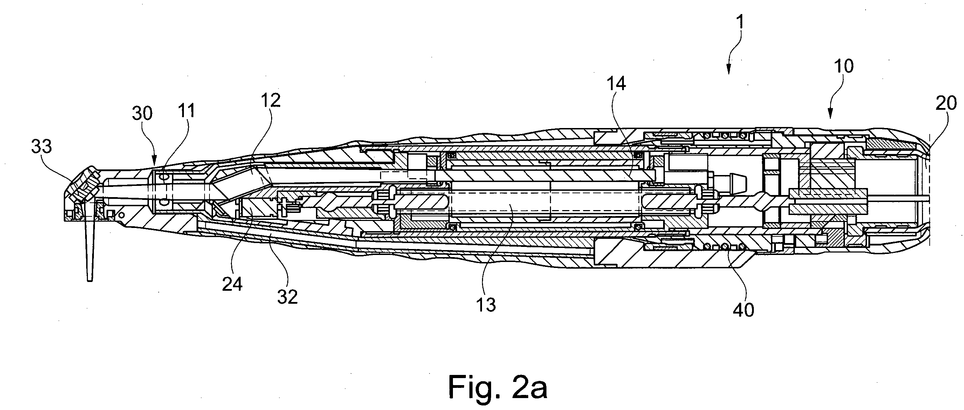

From Fig. 1, and in particular Fig. 2a and 2b, one can take further that the mirror arrangement 33 is provided in the handpiece attachment such that it is arranged in the center axis of the tubular interior cavity 31. Since this center axis of the cavity 31 of the handpiece attachment is also essentially the rotational axis thereof and the handpiece attachment 30 is adapted to be rotatable relative to the handpiece body 10, it is possible to position the attachment 30 in different angular working orientations, as described above.

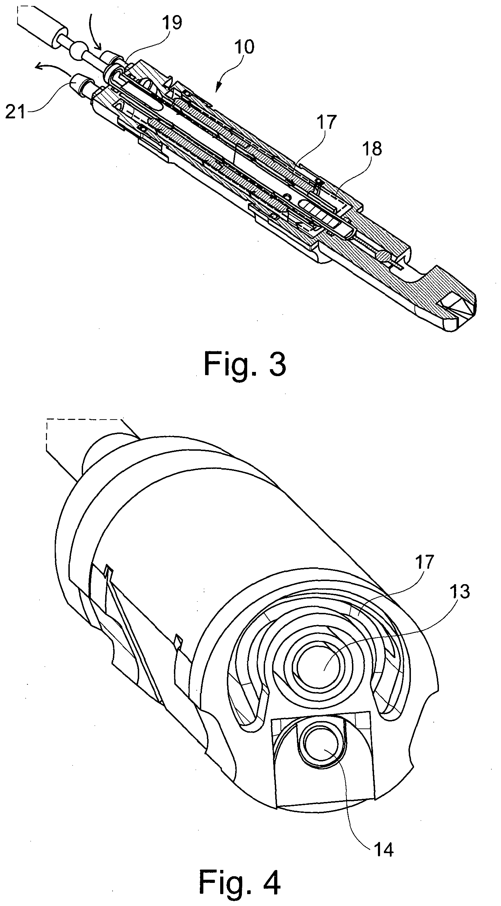

The handpiece body 10 accommodates the laser, and in particular a laser rod 14, as for example Nd:YAG or Er:YAG. Arranged parallel to the laser rod 14, a flash lamp 13 is arranged and both are arranged together in a laser pumping chamber 17 (confer to Fig. 3 and 4). This particular construction is generally known to the skilled person, and it is therefore refrained from a detailed explanation of the technical details thereof.

In addition to the main or working laser beam provided by flash 13 and laser rod 14, optionally a pilot laser 24 is provided at the tip end of handpiece body 10. Since the working laser is preferably an IR-Laser beam, which is not visible to the human eye, the pilot laser beam emitted by pilot laser 24 emits a beam in the visible light range to allow an operator to precisely direct the main or working laser beam. Therefore, the laser beam of pilot laser 24 is likewise imaged through prism 12 and lens 11 and directed to mirror arrangement 33 to combine with the main or working (therapeutic) laser beam. In Fig. 2b one can further see two spray nozzles 34 provided in close proximity to the exit of the laser beam as well as the corresponding coolant tubing 32 provided in attachment 30 and the supply conduit 16 provided in handpiece body 10, all being in fluid communication with each other.

Now referring in particular to Fig. 3 and 4, one can further take that the handpiece 10 comprises a coolant water channel 18 to effectively cool the inner and the outer walls of the pumping chamber 17. To this end, the handpiece 10 comprises a cooling entrance 19 and cooling exit 21.

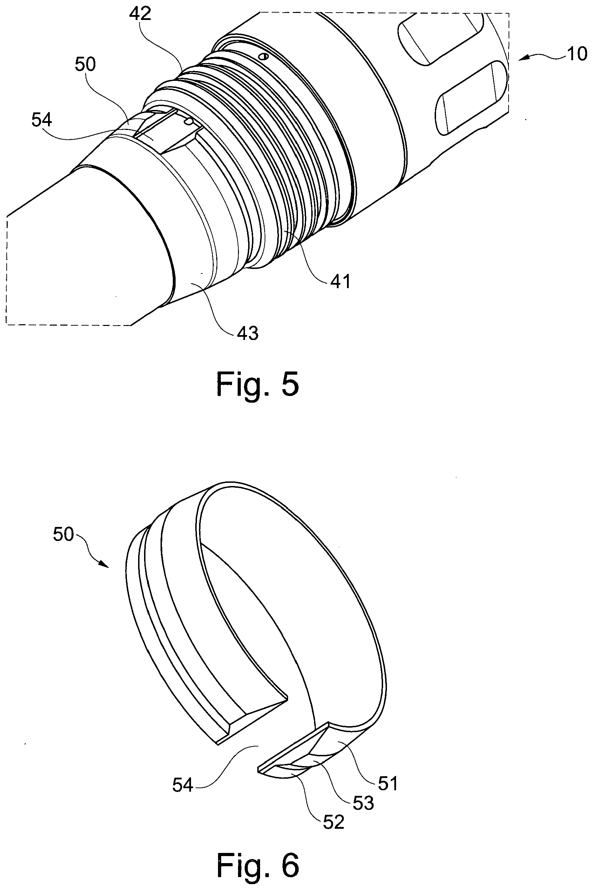

In Fig. 5, a three dimensional schematic illustration of the details of the latch type fixation mechanism 40 is shown. The handpiece body 10 comprises a plurality of annular grooves 42. In the shown embodiment the handpiece body 10 has exemplarily five annular groves. In three of these annular grooves 42, sealing rings 41 are arranged. The annular grooves 42 thus serve for one the purpose to receive the sealing rings 41 therein, but also to supply coolant from the handpiece body to the handpiece attachment, irrespective of the angular or rotational orientation of handpiece attachment to handpiece body, as will be explained in more detail down below.

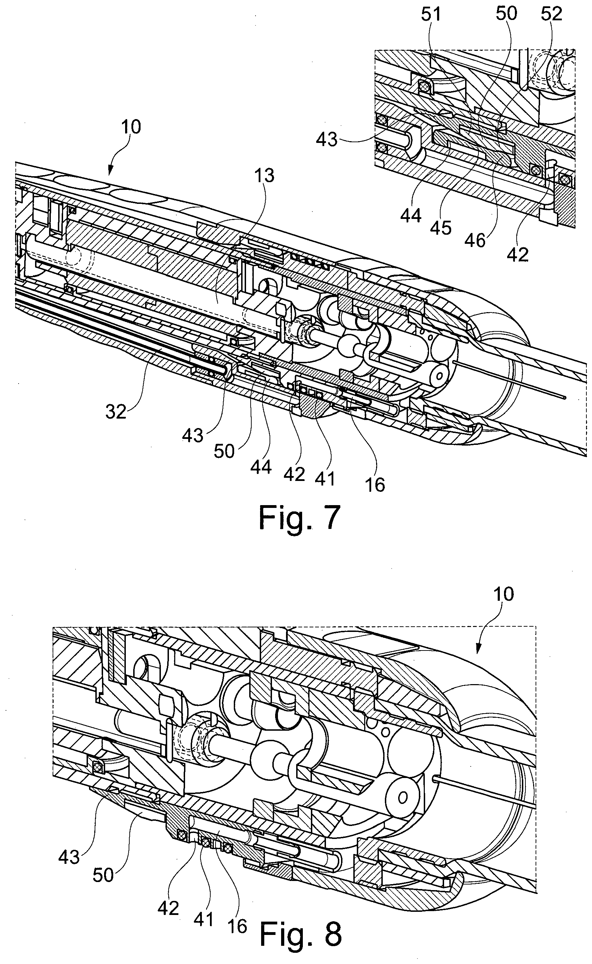

Further, attached to handpiece body 10 is a spring element 50, which is shown in a detailed view in Fig. 6. The spring element 50 is preferably a C-ring spring element as shown in the figures. As the skilled person will recognize, the spring element 50 is adapted to provide a radial spring bias, i.e. it can be compressed radial inwardly to some extend and will provide a corresponding biasing force radial outwardly. Thereby, it can latch into a corresponding annular latching recess provided on the interior wall of handpiece attachment 30. The spring element 50 is held in longitudinal direction on handpiece body 10 due to stop member 43, as can more clearly be seen in Figs. 7 and 8. The shown spring element 50 is a c-spring having an inclined surface 51, a locking recess 52 and a locking ridge 53. All the surfaces are annular on the outer surface of spring element 50. As the skilled person will recognize, spring element 50 can be radially compressed to some extent, due to gap 54, to allow a releasable fixation of handpiece attachment to handpiece body.

Now turning to Fig. 7, one can see how handpiece attachment 30 is fixed onto handpiece body 10. Fixed in the entrance of the interior cavity of handpiece attachment 30, a latching nut 44 is provided. Referring to the enlarged detail of Fig. 7, the latching nut 44 comprises an inner, annular locking recess 45 (latching recess), which is adapted to interact with the locking ridge 53 of spring element 50, and also an annular locking projection 46 which is adapted to interact with the locking recess 52 of spring 50. Latching nut 44 is a separate part to facilitate the assembly of attachment 30, but is can likewise also be integrally formed with attachment 30, i.e. the locking recess 45 and locking projection 46 could e.g. be part of the inner wall of the handpiece attachment. Upon assembly, the latching nut 44 is pushed over the inclined surface 51 of spring element 50, thereby radially pressing spring element 50 together until locking projection 46 latches behind locking ridge 53 into locking recess 52 of spring element 50. Further, spring element 50 springs back into its original shape (as shown in Fig. 7) so that locking ridge 53 latches into the locking recess 45 of nut 44, thereby fixing handpiece attachment 30 and handpiece body 10 together. The skilled person will recognize, that this kind of fixation allows to rotate handpiece attachment 30 relative to handpiece body 10 in the assembled condition shown in Fig. 7.

From Fig. 7 and 8 one can also derive how the coolant supply from the hand piece body 10 to the hand piece attachment 30 is realized in the preferred embodiment. In Fig. 8, one can see a supply conduit 16 provided in the handpiece body 10. The supply conduit 16 exits into one of the annular grooves 42, which is not provided with a sealing ring. The removable handpiece attachment 30 comprises a tubing 32, which is in fluid communication with the annular groove 42, which opens to supply conduit 16. In other words, annular groove 42 is in fluid communication with both the conduit 16 and the tubing 32. Thus, it is possible to arrange handpiece attachment 30 in any angular relation to handpiece body 10 without interrupting the coolant supply from body 10 to attachment 30, since the annular groove 42 provided with coolant from supply conduit 16 can distribute the coolant to any point of annular groove 42. From Figs. 7 and 8 one can further see how the spring element 50 is held in longitudinal direction by a corresponding shape of the handpiece body 10 and in particular by the stop member 43. The stop member 43 has an inclined surface, so that it is easily possible to slit spring element 50 over that inclination to bring it in the position shown in Figs. 7 and 8.

## Claims

1. Handheld laser device (1) for medical, in particular dental treatment purposes, comprising a handpiece having a handpiece body (10) accommodating a laser and a removable handpiece attachment (30), wherein the handpiece attachment (30) is slidable over at least a major portion of the handpiece body (10) and whereby air and/or water is supplied from the handpiece body (10) to the handpiece attachment (30), and
wherein a ring shaped spring element (50) is assigned to the handpiece body (10), in particular a c-ring spring element, offering a radial bias to provide a latch-type fixation for coupling the handpiece body (10) and the handpiece attachment (30).

2. Handheld laser device according to claim 1, whereby the handpiece attachment (30) is adapted to be rotatable relative to the handpiece body, to allow at least two different angular working orientations.

3. Handheld laser device according to claim 1, wherein the ring shaped element (50) is arranged on the handpiece body and comprises an annular locking ridge (53) on its outer surface and the handpiece attachment (30) is slidable over said annular locking ridge (53) and comprises a corresponding annular locking recess (45) to interact with said annular locking ridge (53) and/or wherein the ring shaped element (50) is arranged on the handpiece body and comprises an annular locking recess (52) on its outer surface and the handpiece attachment (30) comprises a corresponding annular locking projection (46) to interact with said annular locking recess (52).

4. Handheld laser device according to any one of the preceding claims, wherein the handpiece body (10) comprises an annular groove (42) and a supply conduit (16) for air and/or water; and the handpiece attachment (30) comprises a tubing (32); and wherein said annular groove (42) is in fluid communication with both of said supply conduit (16) for air and/or water and said tubing (32).

5. Handheld laser device according to any one of the preceding claims, wherein the handpiece body (10) has an elongated tubular form and comprises a focusing lens (11) at its tip end adapted to interact with a mirror arrangement (33) provided in the handpiece attachment (30).

6. Handheld laser device according to claim 5, wherein the handpiece attachment (30) comprises a corresponding elongated tubular interior cavity (31) adapted to coaxially receive said major portion of the handpiece body (10) therein.

7. Handheld laser device according to claim 5 or 6, wherein the handpiece body (10) comprises a prism (12) arranged adjacent to and being adapted to cooperating with said focusing lens (11), to guide the laser beam onto said mirror arrangement (33).

8. Handheld laser device according to claim 7, wherein the mirror arrangement (33) provided in the handpiece attachment (30) is arranged in the center axis of the tubular interior cavity (31) of the handpiece attachment (30).

9. Handheld laser device according to claim 8, wherein the center axis of the tubular interior cavity (31) of the handpiece attachment (30) is a rotational axis of the handpiece attachment (30).

10. Handheld laser device according to any one of the preceding claims, wherein the handpiece attachment (30) does not comprise any electrical or electronic equipment.

11. Handheld laser device according to any one of the preceding claims, wherein the handpiece attachment (30) is adapted to be rotatable relative to the handpiece body in coupled condition of handpiece body (10) and handpiece attachment (30).

12. Handheld laser device according to any one of the preceding claims, wherein the laser comprises a laser rod (14), e.g. Nd:YAG or Er:YAG, and wherein preferably reflective coatings are applied to the end faces of the laser rod, thereby forming a laser resonator.

13. Handheld laser device according to any one of the preceding claims, wherein the optical axis of the laser is parallel to the rotational axis of the handpiece attachment.

14. Handheld laser device according to any of the preceding claims, comprising a second laser (24), namely a pilot laser emitting a laser beam in the visible light range, which pilot laser is arranged in the handpiece body (10).

## Patentansprüche

1. Eine handgeführte Laservorrichtung (1) für medizinische, insbesondere zahnärztliche Behandlungszwecke, umfassend ein Handstück, das ein Handstückkörper (10) aufweist, das einen Laser aufnimmt und einem entfernbaren Handstückaufsatz (30),
wobei der Handstückaufsatz (30) über mindestens einen Hauptteil des Handstückkörpers (10) verschiebbar ist und wobei dem Handstückaufsatz (30) Luft und / oder Wasser vom Handstückkörper (10) zugeführt wird, und
wobei dem Handstückkörper (10) ein ringförmiges Federelement (50), insbesondere ein C-Ring-Federelement, zugeordnet ist, das eine radiale Vorspannung zum Vorsehen einer klinkenartigen Fixierung zum Verkuppeln des Handstückkörpers (10) und des Handstückaufsatzes (30) bereitstellt.

2. Die handgeführte Laservorrichtung nach Anspruch 1, wobei der Handstückaufsatz (30) relativ zu dem Handstückkörper drehbar ausgebildet ist, um mindestens zwei unterschiedliche Winkelausrichtungen zu ermöglichen.

3. Die handgeführte Laservorrichtung nach Anspruch 1, wobei das ringförmige Element (50) am Handstückkörper angeordnet ist und an seiner Außenfläche einen ringförmigen Verriegelungssteg (53) aufweist und der Handstückaufsatz (30) über die ringförmige Verriegelung (53) gleiten kann und eine entsprechende ringförmige Verriegelungsvertiefung (45) aufweist, um mit der ringförmigen Verriegelungsrippe (53) zu interagieren und / oder
wobei das ringförmige Element (50) an dem Handstückkörper angeordnet ist und an seiner Außenfläche eine ringförmige Verriegelungsvertiefung (52) aufweist und der Handstückaufsatz (30) einen entsprechenden ringförmigen Verriegelungsvorsprung (46) zum Interagieren mit der ringförmigen Verriegelungsvertiefung (52) aufweist.

4. Die handgeführte Laservorrichtung nach einem der vorhergehenden Ansprüche,
wobei der Handstückkörper (10) eine ringförmige Nut (42) und eine Zuführleitung (16) für Luft und / oder Wasser aufweist; und
der Handstückaufsatz (30) eine Rohrleitung (32) aufweist; und
wobei die ringförmige Nut (42) in einer Flüssigkeitsübertragung sowohl mit der Zuführleitung (16) für Luft und / oder Wasser als auch mit dem Rohrleitung (32) steht.

5. Die handgeführte Laservorrichtung nach einem der vorhergehenden Ansprüche, wobei der Handstückkörper (10) eine längliche röhrenförmige Form aufweist und an seinem vorderen Ende eine Fokussierlinse (11) aufweist, die mit einer im Handstückaufsatz (30) vorgesehenen Spiegelanordnung (33) interagieren kann.

6. Die handgeführte Laservorrichtung nach Anspruch 5, wobei der Handstückaufsatz (30) einen entsprechenden länglichen röhrenförmige inneren Hohlraum (31) aufweist, der dazu ausgelegt ist, den Hauptteil des Handstückkörpers (10) koaxial darin aufzunehmen.

7. Die handgeführte Laservorrichtung nach Anspruch 5 oder 6, wobei der Handstückkörper (10) ein Prisma (12) umfasst, das angrenzend zu der Fokussierlinse (11) angeordnet ist und mit dieser zusammenwirken kann, um den Laserstrahl auf die genannte Spiegelanordnung (33) zu leiten.

8. Die handgeführte Laservorrichtung nach Anspruch 7, wobei die im Handstückaufsatz (30) vorgesehene Spiegelanordnung (33) in der Mittelachse des röhrenförmigen inneren Hohlraums (31) des Handstückaufsatzes (30) angeordnet ist.

9. Die handgeführte Laservorrichtung nach Anspruch 8, wobei die Mittelachse des röhrenförmigen inneren Hohlraums (31) des Handstückaufsatzes (30) eine Drehachse des Handstückaufsatzes (30) ist.

10. Die handgeführte Laservorrichtung nach einem der vorhergehenden Ansprüche, wobei der Handstückaufsatz (30) kein elektrisches oder elektronisches Equipment umfasst.

11. Die handgeführte Laservorrichtung nach einem der vorhergehenden Ansprüche, wobei der Handstückaufsatz (30) relativ zu dem Handstückkörper im gekuppelten Zustand des Handstückkörpers (10) und des Handstückaufsatzes (30) drehbar ist.

12. Die handgeführte Laservorrichtung nach einem der vorhergehenden Ansprüche, wobei der Laser einen Laserstab (14), z. Nd: YAG oder Er: YAG und wobei vorzugsweise reflektierende Beschichtungen auf die Endflächen des Laserstabs aufgebracht werden, wodurch ein Laserresonator gebildet wird.

13. Die handgeführte Laservorrichtung nach einem der vorhergehenden Ansprüche, wobei die optische Achse des Lasers parallel zur Rotationsachse des Handstückaufsatzes ist.

14. Die handgeführte Laservorrichtung nach einem der vorhergehenden Ansprüche, umfassend einen zweiten Laser (24), nämlich einem Pilotlaser, der einen Laserstrahl im Bereich des sichtbaren Lichts emittiert, wobei der Pilotlaser im Handstückkörper (10) angeordnet ist.

## Revendications

1. Dispositif laser tenu à la main (1) pour des fins médicales, en particulier pour traitement dentaire, possédant un corps de poignée (10) logeant un laser et un accessoire amovible de poignée (30),
dans lequel l'accessoire de poignée (30) peut coulisser au-dessus d'au moins une majeure partie du corps de poignée (10) et de manière que de l'air et/ou de l'eau soit délivré depuis le corps de poignée (10) à l'accessoire de poignée (30), et
dans lequel un élément ressort de forme annulaire (50) est adjoint au corps de poignée (10), en particulier un élément ressort annulaire en C, procurant une sollicitation radiale pour assurer une fixation de type verrouillable pour le couplage du corps de poignée (10) et de l'accessoire de poignée (30).

2. Dispositif laser tenu à la main selon la revendication 1, tel que l'accessoire de poignée (30) soit adapté pour pouvoir tourner par rapport au corps de poignée, pour autoriser au moins deux orientations angulaires de travail différentes.

3. Dispositif laser tenu à la main selon la revendication 1, dans lequel l'élément de forme annulaire (50) est agencé sur le corps de poignée et comprend une nervure de verrouillage annulaire (53) sur sa surface externe et l'accessoire de poignée (30) peut coulisser au-dessus de ladite nervure de verrouillage annulaire (53) et comprend un creux de verrouillage annulaire correspondant (45) destiné à interagir avec ladite nervure de verrouillage annulaire (53) et/ou dans lequel l'élément de forme annulaire (50) est agencé sur le corps de poignée et comprend un creux de verrouillage annulaire (52) sur sa surface extérieure et l'accessoire de poignée (30) comprend une saillie de verrouillage annulaire correspondante (46) destinée à interagir avec ledit creux de verrouillage annulaire (52).

4. Dispositif laser tenu à la main selon l'une des revendications précédentes, dans lequel le corps de poignée (10) comprend une gorge annulaire (42) et une conduite d'alimentation (16) pour de l'air et/ou de l'eau; et l'accessoire de poignée (30) comprend une tubulure (32) ; et dans lequel ladite gorge annulaire (42) est en communication de fluide avec à la fois ladite conduite d'alimentation (16) pour de l'air et/ou de l'eau et ladite tubulure (32).

5. Dispositif laser tenu à la main selon l'une des revendications précédentes, dans lequel le corps de poignée (10) présente une forme tubulaire allongée et comprend une lentille de focalisation (11) à son bout extrême, apte à interagir avec un agencement de miroir (33) disposé dans l'accessoire de poignée (30).

6. Dispositif laser tenu à la main selon la revendication 5, dans lequel l'accessoire de poignée (30) comprend une cavité intérieure tubulaire allongée correspondante (31) apte à recevoir coaxialement à l'intérieur ladite majeure portion du corps de poignée (10).

7. Dispositif laser tenu à la main selon la revendication 5 ou 6, dans lequel le corps de poignée (10) comprend un prisme (12) agencé adjacent à, et étant adapté pour coopérer avec, ladite lentille de focalisation (11), pour guider le faisceau laser jusque sur ledit agencement de miroir (33).

8. Dispositif laser tenu à la main selon la revendication 7, dans lequel l'agencement de miroir (33) disposé dans l'accessoire de poignée (30) est agencé dans l'axe central de la cavité intérieure tubulaire (31) de l'accessoire de poignée (30).

9. Dispositif laser tenu à la main selon la revendication 8, dans lequel l'axe central de la cavité intérieure tubulaire (31) de l'accessoire de poignée (30) est un axe de rotation de l'accessoire de poignée (30).

10. Dispositif laser tenu à la main selon l'une des revendications précédentes, dans lequel l'accessoire de poignée (30) ne comprend aucun équipement électrique ou électronique.

11. Dispositif laser tenu à la main selon l'une des revendications précédentes, dans lequel l'accessoire de poignée (30) est apte à être tourné par rapport au corps de poignée dans un état de couplage du corps de poignée (10) et de l'accessoire de poignée (30).

12. Dispositif laser tenu à la main selon l'une des revendications précédentes, dans lequel le laser comprend un barreau laser (14), par exemple Nd:YAG ou Er:YAG, et dans lequel des revêtements réflecteurs sont de préférence appliqués sur les faces d'extrémité du barreau laser, pour former ainsi un résonateur laser.

13. Dispositif laser tenu à la main selon l'une des revendications précédentes, dans lequel l'axe optique du laser est parallèle à l'axe de rotation de l'accessoire de poignée.

14. Dispositif laser tenu à la main selon l'une des revendications précédentes, comprenant un second laser (24), à savoir un laser pilote émettant un faisceau laser dans la plage de lumière lisible, ce laser pilote étant agencé dans le corps de poignée (10).
